(19) **Europäisches Patentamt / European Patent Office / Office européen des brevets**

(11) **EP 3 384 843 B1**

(12) **EUROPEAN PATENT SPECIFICATION**

(45) Date of publication and mention
of the grant of the patent:
**30.07.2025   Bulletin 2025/31**

(21) Application number: **17164914.8**

(22) Date of filing: **05.04.2017**

(51) International Patent Classification (IPC):
*A61B 5/0507* (2021.01)    *A61B 5/08* (2006.01)
*A61B 5/16* (2006.01)

(52) Cooperative Patent Classification (CPC):
**A61B 5/165; A61B 5/0507; A61B 5/0816**

(54) **DETERMINING BREATHING ATTRIBUTES BY A DETECTION DEVICE**

BESTIMMUNG VON ATMUNGSATTRIBUTEN MIT EINER ERKENNUNGSVORRICHTUNG

DÉTERMINATION DES ATTRIBUTS RESPIRATOIRES PAR UN DISPOSITIF DE DÉTECTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**10.10.2018   Bulletin 2018/41**

(73) Proprietor: **Nokia Technologies Oy**
**02610 Espoo (FI)**

(72) Inventors:
• **RAHMAN, MD-Mahbubur**
**Sunnyvale, CA 94086 (US)**
• **BAILEY, Marc**
**Cambridge, CB4 3DD (GB)**
• **SHAMAIN, Durgaprasad**
**San Jose, CA 95154 (US)**

(74) Representative: **Swindell & Pearson Limited**
**48 Friar Gate**
**Derby DE1 1GY (GB)**

(56) References cited:
WO-A1-2014/055755      JP-A- 2008 253 538
JP-A- 2016 107 095       US-A1- 2010 130 873
US-A1- 2010 152 600      US-A1- 2011 060 215

• **CHANGZHAN GU ET AL: "Assessment of Human Respiration Patterns via Noncontact Sensing Using Doppler Multi-Radar System", SENSORS, vol. 15, no. 3, 16 March 2015 (2015-03-16), pages 6383 - 6398, XP055464665, DOI: 10.3390/ s150306383**
• **MASSAGRAM W ET AL: "Assessment of Heart Rate Variability and Respiratory Sinus Arrhythmia via Doppler Radar", IEEE TRANSACTIONS ON MICROWAVE THEORY AND TECHNIQUES, PLENUM, USA, vol. 57, no. 10, 1 October 2009 (2009-10-01), pages 2542 - 2549, XP011276523, ISSN: 0018-9480, DOI: 10.1109/ TMTT.2009.2029716**

EP 3 384 843 B1

**Description**

TECHNOLOGICAL FIELD

An example embodiment of the present disclosure relates generally to interpretation of data collected by a detection device. Some certain example embodiments are directed to a method, apparatus and computer program product for determining a stress recovery based on breathing attributes.

BACKGROUND

In the study of physiological stress analysis, medical professionals often rely on explicit user input regarding their feelings and stress levels. In many instances, a user may need to acknowledge their stress level in order to make improvements to daily activity and to mitigate the stress. In other examples of stress study and the effect of stress on an individual's health, subjects may wear devices such as heart rate monitors. Such devices may be intrusive to the user, and are inconvenient to wear on an everyday basis. Further, it can be difficult to determine whether changes in heart rate, heart rate variability, and the like are attributable to stress, or another factor such as the user's activity.

US2011/060215 (D1) discloses an apparatus and method for non-invasive and continuous measurement of respiratory chamber volume and associated parameters including respiratory rate, respiratory rhythm, tidal volume, dielectric variability and respiratory congestion. In particular, a non-invasive apparatus and method for determining dynamic and structural physiologic data from a living subject including a change in the spatial configuration of a respiratory chamber, a lung or a lobe of a lung to determine overall respiratory health comprising an ultra wide-band radar system having at least one transmitting and receiving antenna for applying ultra wide-band radio signals to a target area of the subject's anatomy wherein the receiving antenna collects and transmits signal returns from the target area.

WO2104/055755 (D2) discloses a sensing system that includes a first radar sensing assembly and an analysis system. According to D2, the first radar sensing assembly measures plural distances to a first target location at different times using radar. In D2, the analysis system receives the plural distances from the first radar sensing assembly and quantifies movements of a target object at the first target location at the different times by calculating differences in the plural distances measured by the first radar sensing assembly. According to D2, the analysis system generates one or more first quantified activity level values indicative of the movements of the target object at the first target location using the differences in the plural distances that are calculated.

US2010/152600 (D3) is a non-contact radar biosignal measurement device disclosing chest vs abdominal breathing phase difference, as well as calibration means, and HRV detection.

BRIEF SUMMARY

A method, apparatus, and computer program product are therefore provided for determining breathing attributes by a detection device and utilizing the breathing attributes to determine stress recovery. Example embodiments may receive a radar signal from a detection device, and extract information describing chest breathing and abdominal breathing. In some examples, the chest and abdominal breathing information may be utilized, along with a stress indicator, to determine whether the user is recovering from the stress. The chest and abdominal breathing information may indicate how effectively the user's breathing reduces physiological stress conditions, such as acute physiological stress conditions.

In particular, example embodiments may determine a stress recovery level by monitoring a phase difference between the chest and abdominal breathing information and associating the phase difference with a stress indicator. The stress indicator may be detected via the detection device and/or other sensors, or received from another apparatus, and may include heart rate variability (HRV), and/or other biometric measurements such as but not limited to temperature, facial recognition, galvanic skin sensing data, and/or the like. In some embodiments, target respiration information may be calibrated based on one or more stress indicators. In this regard, target respiration information may be calibrated for a particular user based on the stress indicator.

In certain example embodiments a breathing exercise may be provided via a user interface to guide a user to relax and/or recover from the stress. For example, if a threshold level of stress is reached, the user may be presented with a breathing exercise. As another example, the exercise may be presented in response to a user input. Feedback may also be provided regarding the efficacy of the breathing exercise in reducing the user's stress.

A method is provided. In examples, the method includes receiving user respiration information according to a radar signal detected from a radar detection device, extracting chest breathing information and abdominal breathing information from the user respiration information, and associating a phase difference between the chest breathing information and abdominal breathing information with a stress level indicator to determine stress recovery. In certain embodiments, the stress level indicator comprises heart rate variability (HRV) and the method further includes calibrating target respiration information based on the HRV information.

The method may further include estimating mouth movement data according to the radar signal, and refining the user respiration information based on the mouth movement data.

In certain examples, the method includes identifying data provided according to the radar signal as attributed to speech. The method may further include comparing the user respiration information to target respiration information. The method may also include, based on the comparison of the user respiration information and the target respiration information, determining a difference between the user respiration information and the target respiration information, and in response to determining the difference, causing provision of feedback via a user interface.

According to some examples, the phase difference between the user respiration information and the target respiration information is determined as indicative of stress recovery. The method may further include identifying target respiration information for a particular user based on previously received user respiration information relating to the particular user. The method may further include determining target respiration information from a library of respiration information.

In certain examples the method may further include causing provision of information relating to a breathing exercise, determining an efficacy of the breathing exercise by monitoring the user respiration information, and causing provision of feedback relating to the efficacy of the breathing exercise.

An apparatus is also provided. in examples, the apparatus comprises at least one signal detection device, at least one processor, and at least one memory including computer program code, the at least one memory and the computer program code configured to, with the processor, cause the apparatus to at least generate user respiration information according to a signal detected from the signal detection device, generate chest breathing information and abdominal breathing information from the use respiration information, and associate a phase difference between the chest breathing information and abdominal breathing information with a stress indicator to determine stress recovery.

In some examples, the signal detection device comprises a radar detection device configured to emit a radar signal with a transmitter, and with a transceiver, detect the radar signal reflected from a body, wherein the user respiration information is generated according to the reflected radar signal. In certain embodiments, the stress level indicator comprises heart rate variability (HRV) and wherein the at least one memory and the computer program code are further configured to calibrate target respiration information based on the HRV information.

In some examples, the at least one memory and the computer program code are further configured to, with the processor, cause the apparatus to at least compare the user respiration information to target respiration information, based on the comparison of the user respiration information and the target respiration information, determine a difference between the user respiration information and the target respiration information, and in response to determining the difference, causing provision of feedback via a user interface.

A computer program product is also provided. In examples, the computer program product comprises at least one non-transitory computer-readable storage medium having computer-executable program code instructions stored therein, the computer-executable program code instructions comprising program code instructions to at least receive user respiration information according to a radar signal detected from a radar detection device, extract chest breathing information and abdominal breathing information from the user respiration information, and

associating a phase difference between the chest breathing information and abdominal breathing information with a stress indicator to determine stress recovery.

The computer-executable program code instructions may comprise program code instructions to at least receive heart rate variability (HRV) information according to the radar signal, and calibrate target respiration information based on the HRV information.

In examples, an apparatus is provided with means for receiving user respiration information according to a radar signal detected from a radar detection device, means for extracting chest breathing information and abdominal breathing information from the user respiration information, and means for associating a phase difference between the chest breathing information and abdominal breathing information with a stress level indicator to determine stress recovery.

According to various, but not necessarily all, embodiments of the invention there is provided a method, an apparatus and a computer program product according to the appended claims.

BRIEF DESCRIPTION OF THE DRAWINGS

Having thus described certain example embodiments of the present disclosure in general terms, reference will hereinafter be made to the accompanying drawings which are not necessarily drawn to scale, and wherein:

Figure 1 is a block diagram of an apparatus that may be configured to implement example embodiments of the present disclosure;
Figure 2 illustrates a radar detection device situated proximate a body;
Figure 3 is a flowchart illustrating operations performed in accordance with example embodiments of the present disclosure;
Figure 4 is a graph of breathing information according to example embodiments;
Figure 5 is a flowchart illustrating operations performed in accordance with example embodiments of the present disclosure ;
Figure 6 is a user interface according to example embodiments;
Figure 7 is a flowchart illustrating operations performed in accordance with example embodiments of the present disclosure ;

Figure 8 is a flowchart illustrating operations performed in accordance with example embodiments of the present disclosure; and

Figure 9 is a flowchart illustrating a flow of data in accordance with example embodiments of the present disclosure.

DETAILED DESCRIPTION

Some embodiments of the present disclosure will now be described more fully hereinafter with reference to the accompanying drawings, in which some, but not all, embodiments of the disclosure are shown. Indeed, various embodiments of the disclosure may be embodied in many different forms and should not be construed as limited to the embodiments set forth herein; rather, these embodiments are provided so that this disclosure will satisfy applicable legal requirements. Like reference numerals refer to like elements throughout. As used herein, the terms "data," "content," "information," and similar terms may be used interchangeably to refer to data capable of being transmitted, received and/or stored in accordance with embodiments of the present disclosure. Thus, use of any such terms should not be taken to limit the scope of embodiments of the present disclosure.

Additionally, as used herein, the term 'circuitry' refers to (a) hardware-only circuit implementations (e.g., implementations in analog circuitry and/or digital circuitry); (b) combinations of circuits and computer program product(s) comprising software and/or firmware instructions stored on one or more computer readable memories that work together to cause an apparatus to perform one or more functions described herein; and (c) circuits, such as, for example, a microprocessor(s) or a portion of a microprocessor(s), that require software or firmware for operation even if the software or firmware is not physically present. This definition of 'circuitry' applies to all uses of this term herein, including in any claims. As a further example, as used herein, the term 'circuitry' also includes an implementation comprising one or more processors and/or portion(s) thereof and accompanying software and/or firmware. As another example, the term 'circuitry' as used herein also includes, for example, a baseband integrated circuit or applications processor integrated circuit for a mobile phone or a similar integrated circuit in a server, a cellular network device, other network device, field programmable gate array, and/or other computing device.

As defined herein, a "computer-readable storage medium," which refers to a physical storage medium (e.g., volatile or non-volatile memory device), may be differentiated from a "computer-readable transmission medium," which refers to an electromagnetic signal.

As described herein, a method, apparatus and computer program product are provided for determining stress recovery based on chest and abdominal breathing information detected with a detection device, such as a radar detection device. The breathing information is associated with a stress indicator, such as HRV, to determine stress recovery. The data collected by the radar detection device may be collected via a mobile or wearable device in the vicinity of a body, but it will be appreciated that the data may be collected and interpreted by any type of application in which the determination of breathing information and stress recovery may be of value according to example embodiments. Although a radar detection device is discussed herein, any detection device having elements configured to determine breathing information and corresponding stress levels using the techniques described herein may be implemented to carry out the techniques described herein. The techniques described herein may provide a technical effect of detecting certain behaviors and/or associated breathing patterns of a user that may increase the effectiveness of activity monitoring and/or breathing exercises in fields such as digital health. Other technical effects are discussed herein.

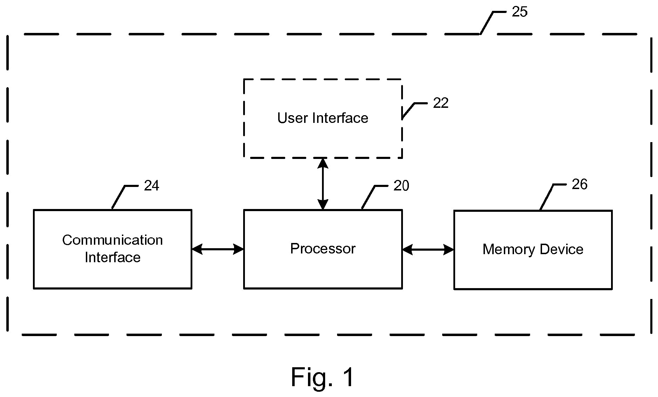

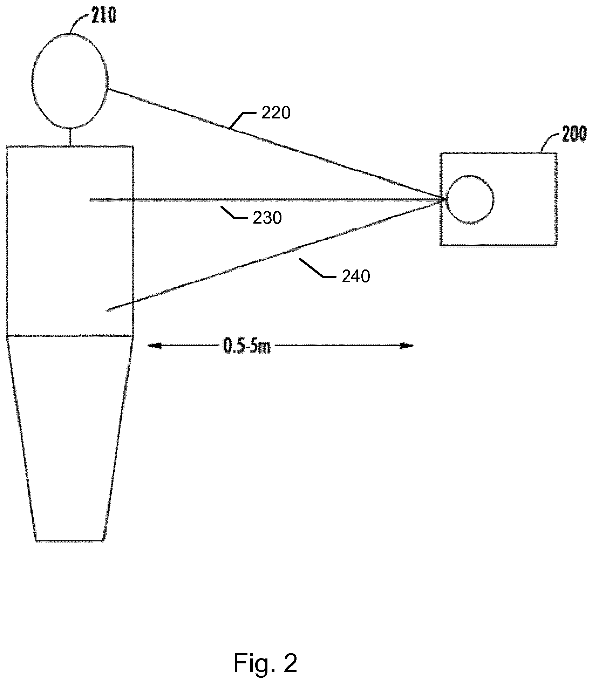

Referring to Figure 1, apparatus 25 may include or otherwise be in communication with a processor 20, communication interface 24, and memory device 26. As described below and as indicated by the dashed lines in Figure 1, in some embodiments, the apparatus 25 may also optionally include a user interface 22. Apparatus 25 may be implemented as a server or distributed system, such as a server for processing respiration data, data collected by a radar detection device and/or the like. In some examples, apparatus 25 need not necessarily be embodied by a server, and may be embodied by a wide variety of devices including personal computers, work stations, or mobile terminals, such as laptop computers, tablet computers, smartphones or any combination of the aforementioned, wearable devices, and other types of voice and text communications systems. In some examples, apparatus 25 may be embodied by or otherwise associated with, (e.g., communicatively connected with), a radar detection device that emits and detects radar signals for subsequent processing. Positioning and use of the radar detection device is described below with respect to Figure 2.

In some embodiments, the processor 20 (and/or co-processors or any other processing circuitry assisting or otherwise associated with the processor 20) may be in communication with the memory device 26 via a bus for passing information among components of the apparatus 25. The memory device 26 may include, for example, one or more volatile and/or non-volatile memories. In other words, for example, the memory device 26 may be an electronic storage device (e.g., a computer readable storage medium) comprising gates configured to store data (e.g., bits) that may be retrievable by a machine (e.g., a computing device like the processor 20). The memory device 26 may be configured to store information, data, content, applications, instructions, or the like for enabling the apparatus to carry out various functions in accordance with an example embodiment of the present disclosure. For example, the memory device 26 could be configured to buffer input data for processing by the processor 20. Additionally or alternatively, the memory device 26 could be configured to store instructions for execution by the processor 20. In some embodiments, the memory device 26 may be configured to store respiration data and/or other data collected and/or converted by a radar detection device.

The apparatus 25 may, in some embodiments, be embodied in various devices as described above (e.g., server, work station, and/or the like). In some embodiments, apparatus 25 may embody the signal detect device (e.g., radar detection device), and the processor 20 may be further configured to implement the functionality described herein in addition to the functions of the signal detection device. However, in some embodiments, the apparatus 25 may be embodied as a chip or chip set. In other words, the apparatus 25 may comprise one or more physical packages (e.g., chips) including materials, components and/or wires on a structural assembly (e.g., a baseboard). The structural assembly may provide physical strength, conservation of size, and/or limitation of electrical interaction for component circuitry included thereon. The apparatus 25 may therefore, in some cases, be configured to implement an embodiment of the present disclosure on a single chip or as a single "system on a chip." As such, in some cases, a chip or chipset may constitute means for performing one or more operations for providing the functionalities described herein.

The processor 20 may be embodied in a number of different ways. For example, the processor 20 may be embodied as one or more of various hardware processing means such as a coprocessor, a microprocessor, a controller, a digital signal processor (DSP), a processing element with or without an accompanying DSP, or various other processing circuitry including integrated circuits such as, for example, an ASIC (application specific integrated circuit), an FPGA (field programmable gate array), a microcontroller unit (MCU), a hardware accelerator, a special-purpose computer chip, or the like. As such, in some embodiments, the processor 20 may include one or more processing cores configured to perform independently. A multi-core processor may enable multiprocessing within a single physical package. Additionally or alternatively, the processor 20 may include one or more processors configured in tandem via the bus to enable independent execution of instructions, pipelining and/or multithreading.

In example embodiments, the processor 20 may be configured to execute instructions stored in the memory device 26 or otherwise accessible to the processor 20. Alternatively or additionally, the processor 20 may be configured to execute hard coded functionality. As such, whether configured by hardware or software methods, or by a combination thereof, the processor 20 may represent an entity (e.g., physically embodied in circuitry) capable of performing operations according to an embodiment of the present disclosure while configured accordingly. Thus, for example, when the processor 20 is embodied as an ASIC, FPGA or the like, the processor 20 may be specifically configured hardware for conducting the operations described herein. Alternatively, as another example, when the processor 20 is embodied as an executor of software instructions, the instructions may specifically configure the processor 20 to perform the algorithms and/or operations described herein when the instructions are executed. However, in some cases, the processor 20 may be a processor of a specific device (e.g., a mobile terminal or network entity) configured to employ an embodiment of the present disclosure by further configuration of the processor 20 by instructions for performing the algorithms and/or operations described herein. The processor 20 may include, among other things, a clock, an arithmetic logic unit (ALU) and logic gates configured to support operation of the processor 20.

Meanwhile, the communication interface 24 may be any means such as a device or circuitry embodied in either hardware or a combination of hardware and software that is configured to receive and/or transmit data from/to a network and/or any other device or module in communication with the apparatus 25. In this regard, the communication interface 24 may include, for example, an antenna (or multiple antennas) and supporting hardware and/or software for enabling communications with a wireless communication network. For example, the communication interface 24 may include an antenna configured to emit or receive radar signals. Additionally or alternatively, the communication interface 24 may include the circuitry for interacting with the antenna(s) to cause transmission of signals via the antenna(s) or to handle receipt of signals received via the antenna(s). In some environments, the communication interface 24 may alternatively or also support wired communication. As such, for example, the communication interface 24 may include a communication modem and/or other hardware/software for supporting communication via cable, digital subscriber line (DSL), universal serial bus (USB) or other mechanisms.

In some embodiments, such as instances in which the apparatus 25 is embodied by a user device, the apparatus 25 may include a user interface 22 that may, in turn, be in communication with the processor 20 to receive an indication of a user input and/or to cause provision of an audible, visual, mechanical or other output to the user. As such, the user interface 22 may include, for example, a keyboard, a mouse, a joystick, a display, a touch screen(s), touch areas, soft keys, a microphone, a speaker, or other input/output mechanisms. Alternatively or additionally, the processor 20 may comprise user interface circuitry configured to control at least some functions of one or more user interface elements such as, for example, a speaker, ringer, microphone, display, and/or the like. The processor 20 and/or user interface circuitry comprising the processor 20 may be configured to control one or more functions of one or more user interface elements through computer program instructions (e.g., software and/or firmware) stored on a memory accessible to the processor 20 (e.g., memory device 26, and/or the like). In some embodiments, the user interface 22 may be configured for feedback to be provided to a user to encourage the user to change the user's respiration rate.

Figure 2 illustrates an example radar detection device 200 situated proximate a body 210 (e.g., the body of a user). The radar detection device 200 used to collect the data processed by apparatus 25 according to example embodiments may be situated in close proximity to a body for which mouth, chest, and/or abdomen movements can be detected. For example, radar detection device 200 may be implemented in a device held in front of the user, or embedded in a workstation and/or monitor in the vicinity of the user's workspace, for example. In certain embodiments, the radar detected device 200 may be implemented in a watch or other wearable device. The radar detection device 200 may be implemented in any suitable device, and may be embodied by and/or communicatively connected by apparatus 25. It will be appreciated that the radar detection device 200 may be considered a radar detection system, and reference to radar detection device or radar detection system used herein is not intended to be limiting.

The radar detection device 200 may be any type of radar detecting unit or system that scans an area, such as approximately .5-5 meters from the body or user. The radar detection device 200 may have a beam that is sufficiently wide so as to cover an area of interest, including the body or any number of bodies. The radar detection device 200 may sweep a radar beam (e.g., radio waves) through the area which may any number of bodies, and may send and receive radar signals. According to the invention, the received radar signals are indicative of mouth movement 220, chest movement 230, and/or abdomen movement 240 as described herein.

In this regard, the radar detection device 200 may include a transmitter for producing electromagnetic waves, and an antenna (either external or internal to the radar detecting device) for emitting the waves. Alternatively, the radar detection device 200 may emit a plurality of pulses. In some examples, the radar detection device 200 may comprise a receiving antenna, which may be embodied within the same or different physical structure as the antenna for emitting the radar signal. In some embodiments, radar detection device 200 may comprise a single antenna. In certain embodiments, however, radar detection device 200 may comprise multiple antennae. In some examples, an antenna may be implemented as a trace on a circuit board that can be used to transmit signals.

The radar detection device 200 may further include a receiver for converting a received radar signal into a format or data that may be processed by apparatus 25. In this regard, the apparatus 25 may process the detected radar signals to perform the operations described herein according to example embodiments. Additionally or alternatively, the detected radar signals may be converted by the radar detection device 200, and/or any other apparatus, to data that is processed by apparatus 25 to perform the operation described herein. In some examples, the radar detection device 200 may include a processor (e.g., processor 20), for processing the data converted by the receiver. In some examples, the processor of the radar detection device 200 may be implemented separately from processor 20.

Radar signals may be emitted from the radar detection device 200 in rapid succession. Detected radar signals received by the radar detecting device may indicate various types of movement of various body parts. The reflected radar signals may therefore be used to determine, monitor, and/or analyze the raising and lowering of the chest and/or abdomen, and/or movement of the mouth and/or neck. In some embodiments, the various movements may be determined from a single antenna and/or detection mechanism.

Radar detection device 200 may be embodied within a same device or housing as apparatus 25. In some examples, radar detection device 200 may be configured to provide data to apparatus 25, such as over a network or by direct wired connection. As yet another example, the radar detection device 200 may provide data to a server or other memory device, and the apparatus 25 may access the data for subsequent processing as described herein and according to example embodiments.

In some cases, the radar detection device 200 provides for non-contact detection of breathing attributes. For example, the radar detection device 200 may be configured to detect breathing when positioned .5 - 5 meters from the body. This range is provided merely as an example and it will be appreciated that the radar detection device 200 may be configured to operate at any distance from the body.

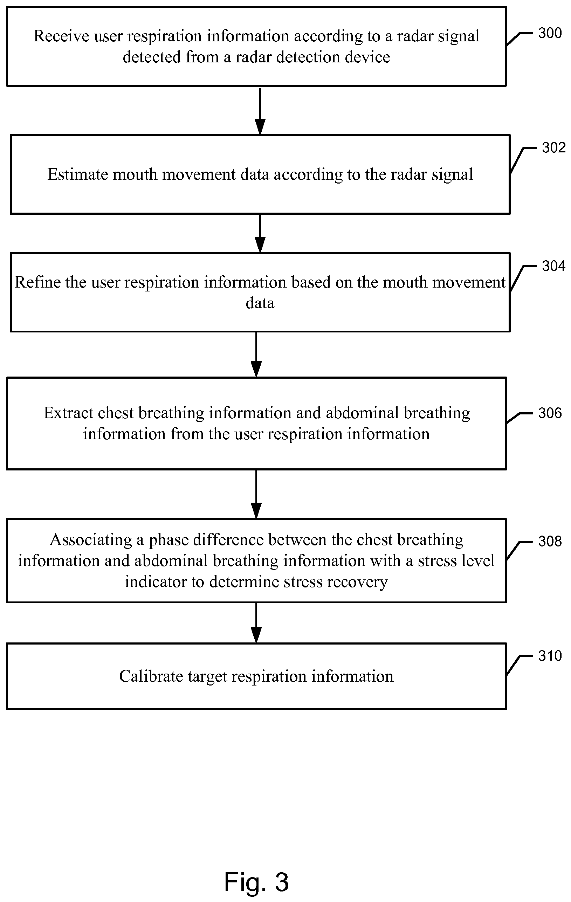

Referring now to Figure 3, the operations for determining a stress recovery level based on chest and abdominal breathing information are outlined in accordance with an example embodiment. In this regard and as described below, the operations of Figure 3 may be performed by apparatus 25. In some cases, the order of Figure 3 may be rearranged to suit various operations of apparatus 25. The operations of Figure 3 may therefore be performed in any order.

As shown by operation 300 in Figure 3, the apparatus 25 may include means, such as the processor 20, communication interface 24, memory device 26, or the like, for receiving user respiration information according to a radar signal detected from a radar detection device. In this regard, the radar detection device 200 may repeatedly emit and detect radar signals. The apparatus 25 may therefore receive or access the data to determine user respiration information.

As described above with respect to Figure 2, a radar detection device 200 may be configured in the vicinity of a body or user. A body or user may therefore refer to any indication or data associated with an individual or body. Reference to the body or user may therefore include any data describing a body, body part, torso, limb and/or extremity, and may further include any type of profile or data detectable by a radar including a heart rate signature, a breathing signature, a height, length, and/or the like.

In some examples, the radar detection system and/or apparatus 25 may differentiate the reflecting source of a signal based on the strength of the detected radar signals. The material or composition of the non-body objects in the environment of the user's body and/or radar detection device 200 may vary from characteristics of bodies such that the radar detection system or apparatus 25 may distinguish between signals reflected from the body and signals reflected from other objects. Detected objects may be identified as bodies based on characteristics consistent with a body. For example, an object having detected measurements falling within a predefined range, may be identified as a body.

The data detected by the radar detection system may describe the movement, positioning, or orientation of a body, person, animal and/or the like. For example, the body may include data describing the positioning of the torso or any body part. The data may therefore reflect the breath of an individual (e.g., movement or raising of the chest and/or abdomen), movement of any body part reflecting a beating heart or pulse, and/or the like. Any data reflecting qualities, characteristics, or measurements relating to breathing data may therefore be referred to herein as user respiration information.

The user respiration information may therefore be generated by apparatus 25 based on the reflected radar signal or associated signal. As another example, the user respiration information may be received by apparatus 25 from a remote device, such as a remote radar detection device or system.

Operations 302 and 304 for estimating mouth movement and refining the data accordingly based on determined speech, as described below, may be considered optional operations and are not necessarily required.

In certain example embodiments, in operation 302, the apparatus 25 the apparatus 25 may include means, such as the processor 20, memory device 26, or the like, for estimating mouth movement data according to the radar signal. The radar detection device 200 may further detect a radar signal reflected from the user's mouth and/or lips, such that the apparatus 25 may determine movements of the mouth are caused by talking, or other forms of communication or mouth movements. As another example, speech or mouth movement may be estimated based on sound detected from a microphone such as may be implemented on user interface 22.

At operation 304, the apparatus 25 may include means, such as the processor 20, memory device 26, or the like, for refining the user respiration information based on the mouth movement data. For example, when the apparatus 25 detects mouth movement or sounds that may be attributable to speech or other communication, the apparatus 25 may determine, such as with processor 20, that the user respiration information having a temporal relationship to the speech (e.g., occurring at or within a threshold timeframe of the mouth movement) is not attributed to stress. Rather, the user respiration information may be attributed to speech and will not be misconstrued as being indicative of stress. In addition to utilizing temporal relationships, apparatus 25 may analyze speech patterns and frequency content. For example, the fricatives have a structure similar to that of white noise like, and vowels have a tonal periodicity. Analysis of speech patterns may therefore further indicate that the user respiration information is attributed to speech and not breathing.

As shown by operation 306, the apparatus 25 may include means, such as the processor 20, the memory device 26, or the like, for extracting chest breathing information and abdominal breathing information from the user respiration information. The chest breathing information and abdominal breathing information may be extracted from a single radar, and may not necessarily require separate radar signals. However, in some embodiments, the chest breathing information and abdominal breathing information may indeed be extracted according to separate radar signals. The apparatus 25 may measure the frequency of a detected radar signal, and detect movements to the chest and/or abdomen within the same signal. The changes in frequency may be referred to as the Doppler effect, the micro Doppler effect, and/or the like. The chest breathing may also be considered as ribcage breathing.

The apparatus 25 may extract chest breathing information and abdominal breathing information based on identifying respective frequencies having a similar pattern to each other. As an individual breathes, their chest and abdomen may expand and detract relatively within unison, such that the similarity in frequencies is significant (although not necessarily identical). For example, a chest breathing frequency may have a phase difference when compared to abdominal breathing frequency.

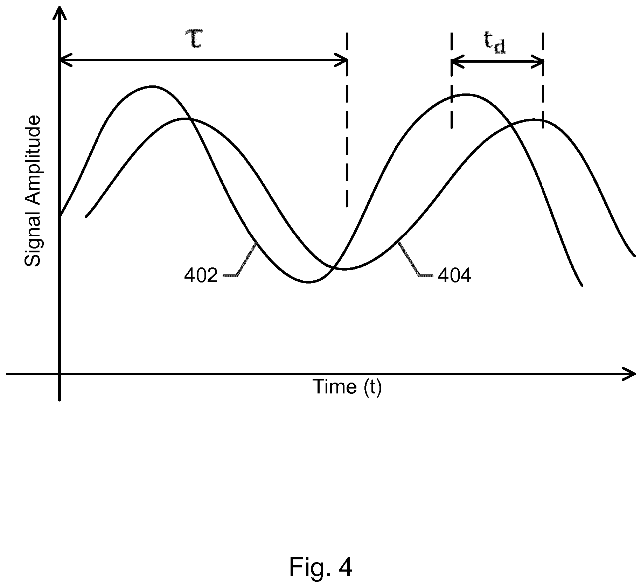

For example, in Figure 4, the relative frequencies of abdominal breathing 402 and or chest breathing 404 are displayed as a function of time. The frequencies 402 and 404 may also be referred to as waveforms. In Figure 4, $t_d$ represents the time difference between consecutive peaks of chest and abdominal wave form. $\tau$ represents the period of the signal or respiration duration.

In certain embodiments, a measurable phase difference between the two signals may be present. The phase difference $\Theta$ between chest and abdominal breathing can be calculated as $\Theta = \dfrac{360°}{\tau} * t_d$. In some examples, the phase difference may change gradually over time. For example, during a relaxation breathing exercise, abdominal breathing may be expected to be more prominent than chest breathing.

In some examples, initial target respiration information may be predefined based on average, standard, or predictable chest and abdomen breathing information. For example, a user may enter user profile information such as gender, age, weight, health conditions, and/or the like, and the apparatus 25 may identify initial target respiration information. The target respiration information may be stored on memory device 26, and/or accessed via a library of respiration information, for example, and may include predefined templates. The captured user respiration information may then be matched or compared to the template breathing information.

As described in further detail below, the apparatus 25 may calibrate target respiration information for a particular user to identify a phase difference that helps the user recover from stress condition. Apparatus 25 may provide user feedback to coach the subject to achieve the optimal phase difference and provide for best stress recovery, as also described in further detail hereinafter.

To differentiate chest breathing from abdominal breathing based on the user respiration information according to a radar signal (including but not limited to a single radar), apparatus 25, such as with processor 20, may associate various frequencies in the signal to various positions of the reflecting source (e.g., the portion of the body from which the signal was reflected) relative to the radar detection device 200. For example, the radar signal reflected from the chest may originate from a position 8-12 inches higher on the body of a user than the radar signal reflected from the abdomen. As another example, a radar detection device 200 positioned at the face level may detect signals reflected from the chest area first (at a closer range) than signals reflected from the abdomen area. In some embodiments, the radar detection device 200 may utilize directional antennas configured to respectively detect signals reflected from the chest are and abdomen area. The apparatus may therefore distinguish data associated with chest movements from data associated with abdomen movements. Calibrations and/or adjustments to account for the position and/or orientation of the device relative to the position and/or orientation of the body may also be made.

As described above, the apparatus 25 may therefore convert the detected radar signals, such as those detected by radar detection device 200, to user respiration information. For example, a user respiration record may comprise data captured and extracted from radar signals detected over a period of time such as several days or month. The apparatus 25 may compile the data associated with one or more users. In some examples, apparatus 25 may store the data on memory device 26 in association with an identifier of an individual user. In some examples, the apparatus 25 may compile the user respiration information record prior to, during or after performing any of the operations described herein.

Apparatus 25 may therefore more recognize changes in breathing relative to a user's standard or expected respiration patterns or characteristics.

As shown by operation 308, the apparatus 25 may include means, such as the processor 20, the memory device 26, or the like, for associating a phase difference between the chest breathing information and abdominal breathing information with a stress indicator to determine stress recovery.

As described above, the phase difference between the chest breathing information and the abdominal breathing information may vary depending upon the particular user, and the state of relaxation or stress. For many users, chest breathing becomes more prominent while under stress than abdominal breathing. In this case, according to the signals, the amplitude of the chest breathing may be larger than the amplitude of the abdominal breathing, despite similar or same frequencies. Abdominal breathing may become more prominent than chest breathing while a user attempts to recover from stress, such as when an individual is taking deep breaths to recover from stress.

The stress indicator may further depend on any data indicative of the user's stress. The stress indicator may therefore include biometric data such as but not limited to heart rate variability (HRV), temperature, galvanic skin sensing data, and/or the like. As another example, facial recognition may be utilized in analyzing facial expressions and deducing stress accordingly.

According to the invention, the apparatus 25 may include means, such as the processor 20, communication interface 24, or the like, for receiving the stress indicator and/or data utilized in determining the stress indicator. For example, the heart rate variability may be detected by the radar detection device 200 and/or by another device or apparatus. Small pulses of the body may be detected by radar signal and extracted from the signal as heart rate information.

The stress indicator may include a quantitative measurement which may be monitored over time. For example, a stress indicator may be calculated as a frequency domain HRV, e.g., low frequency (LF) energy /high frequency (HF) energy ratio of Inter-Beat-Interval (IBI) over a certain time window (e.g., 5 seconds). As another example, the stress indicator may be calculated as a time domain HRV computed by variance of heart rate (or Inter-Beat-Interval) over a certain time window (e.g., 5 seconds). The LF and HF ranges may be predetermined or preconfigured on apparatus 25, or may be otherwise accessible by apparatus 25. For example, the LF component of HRV may be considered between 0.09-0.15 Hz frequency range of IBI, and the HF component may be considered between 0.16- 0.4 Hz frequency range of IBI.

Example embodiments may associate the phase difference with the stress indicator, such as the HRV information, to determine stress recovery. The phase difference between chest breathing information and the abdominal breathing information may be monitored relative to the stress indicator to determine if the user's breathing is indicative of stress recovery. The monitoring of the phase difference relative to the stress indicator may occur over a period of time to track progress in recovering from stress.

Example embodiments may identify a stressful situation by comparing the current HRV with the baseline HRV of a subject. In an example embodiment, apparatus 25 calculates a baseline HRV by averaging the HRV captured during the resting period of a day or at other times of day. Example embodiments may then monitor the HRV along with the user respiration information.

According to some embodiments, a user may attempt abdominal breathing to reduce stress symptoms. A person under stress may take quick and shallow breaths using their chest rather than their diaphragm (e.g., abdomen) to move air in and out of the lung. In such a situation, the phase difference may be smaller (e.g., zero or close to zero). This kind of breathing may disrupt the balances of oxygen and carbon dioxide level in the body. Shallow over-breathing or hyperventilation may prolong the feeling of anxiety by making the physical symptoms of stress worse. Breathing vigorously using the abdomen may help reduce those stress symptoms.

Example embodiments may therefore measure the phase difference between abdominal breathing and chest breathing and compare it to the stress indicator (e.g., HRV) before, during, and/or after the breathing effort, and over a predefined period of time (e.g., 5 minutes). Phase differences in the user respiration information that lead to stress reduction may therefore be identified.

At operation 310, the apparatus 25 may include means, such as the processor 20, the memory device 26, or the like, for calibrating target respiration information. The target respiration information may include target chest breathing information, target abdomen breathing information, and a target phase difference between the chest and abdomen breathing. In some examples, the target phase difference may include a range of phase differences.

According to the invention, the target respiration may be identified and/or calibrated based on the stress indicator of the user, or any other information describing the user. For example, based on the detected or received stress indicators of the particular user, the initially identified target respiration information may be calibrated for the particular user. Any of the target chest breathing information, abdomen breath information, and/or target phase difference may be calibrated based on the stress indicator (e.g., HRV information) of the particular user. Example embodiments may calibrate the target respiration information by determining a user specific range of the phase difference that successfully reduces the user stress.

In some examples, an optimal phase offset (difference) between the abdominal and the chest breathing, which results in reduced stress (e.g., stress recovery), may depend on the body geometry and body composition. Therefore, different people may have different optimal phase offsets. This user-specific phase difference, or more specifically a determined optimal phase-difference range for a user, is estimated during the calibration process.

According to some embodiments, HRV may be considered inversely proportionate to the stress level. For example, an increase in HRV value may indicate a decrease in stress. At time $t$, example embodiments may measure HRV(t) and the phase difference $\theta(t)$.

According to an example embodiment, a user may perform a breathing exercise for T period of time. During this period T, there may be several breathing cycles. For example, if T=5 minutes and the user has an average breathing rate of 15 breaths-per-minute, there will be around 75 breathing cycles on average. Therefore, example embodiments may calculate 75 phase differences $\theta(t)$. Example embodiments may calculate:

$$\theta min = min \ \{I(t) \times \theta(t)\},$$

where $1 < t < T$

$$\theta max = max \ \{I(t) \times \theta(t)\},$$

where $1 < t < T$

where $I(t) = 1$ *if* HRV (t) > HRV (t-1) *or* I(t) = 0, otherwise

Example embodiments may then determine the range of phase difference for a user to recover from stress as $\theta = [\theta min , \theta max]$. The range may therefore represents the calibrating target breathing information for the user. Example embodiments may therefore learn, adjust and/or calibrate the target breathing information based on monitoring of the user's breathing relative to the stress indicator. Example embodiments may particularly learn optimal phase differences by monitoring breathing when the user experiences an acute stress situation and performs a breathing exercise.

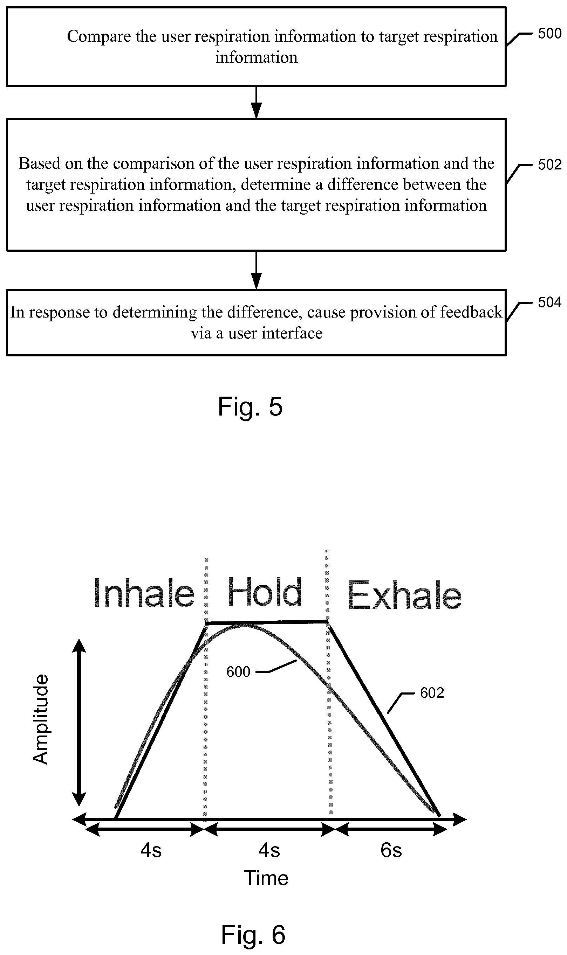

Based on calibrated target breathing information, (which, according to some example embodiments, may be continuously calibrated), apparatus 25 may measure breathing exercise conformance and/or efficacy, as described below with respect to Figure 5.

Figure 5 is a flowchart illustrating operations performed in accordance with example embodiments of the present disclosure. In certain embodiments, the operations of Figure 5 may be performed to provide feedback to a user regarding the user's breathing and/or stress. In this regard, a user may be alerted when their breathing is indicative of stress, and may be further encouraged to mitigate the stress by slowing their breathing.

At operation 500, the apparatus 25 may include means, such as the processor 20, memory device 26, or the like, for comparing the user respiration information to target respiration information. In some embodiments, the apparatus 25 may continually monitor the user respiration information and compare it to target respiration information, which may in some examples, be calibrated or tailored for the particular user. In some examples, the comparison may occur in the background while the user utilizes a user interface for other purposes, or is merely in the vicinity of a user device and/or apparatus 25 and is not necessarily using the device. For example, radar detection device 200 may be implemented in a device held in front of the user, or embedded in a workstation and/or monitor in the vicinity of the user's workspace.

In operation 502, the apparatus 25 may include means, such as the processor 20, memory device 26, or the like, for based on the comparison of the user respiration information and the target respiration information, determining a difference between the user respiration information and the target respiration information. In some embodiments, a difference between the user respiration information and the target respiration information may be indicative of non-compliance with the expected respiration pattern.

In some example embodiments, breathing exercise conformance may be measured by monitoring how closely a user performs abdominal breathing in such a way that the phase difference between the chest and abdominal breathing falls inside an optimal range as defined by the calibrated target breathing information described with respect to operation 306. If apparatus 25 determines the user is performing the exercise accordingly or the phase difference falls within the calculated optimal range [$\theta$ min , $\theta$ max] and I(t) is zero or remains zero, (e.g., HRV (t) $\leq$ HRV (t-1)) as discussed with respect to operation 310, then it will indicate that the user is not recovering and that the breathing exercise may be ineffective. As such, example embodiments may recommend another breathing exercise, or a different kind of exercise, such as but not limited to taking few steps, sipping water, and/or the like.

In operation 504, the apparatus 25 may include means, such as the processor 20, memory device 26, or the like, for in response to determining the difference, causing provision of feedback via a user interface. As described above, if the breathing exercise is ineffective, example embodiments may provide feedback indicative of another breathing exercise, and/or a different kind of exercise.

In some examples, the difference between the user respiration information and the target respiration information may be compared to or measured relative to a threshold difference. For example, if the chest breathing information, abdomen breathing information, and/or phase difference is within a predefined threshold of the associated target respiration information (e.g., target respiration for a particular user), apparatus 25 may prevent feedback regarding stress and/or breathing from being provided. In this scenario, the user may be unaware that the apparatus 25 is monitoring the user's stress and/or breathing. The user may continue to use the device while apparatus 25 monitors the stress and/or breathing. However, if the predefined threshold of difference between the user respiration information and the target respiration information is reached or detected, feedback may be provided via a user interface, such as user interface 22.

In some examples, an alert or other indication may be provided to the user. In some embodiments, apparatus 25 may identify a breathing exercise to assist the user in steadying or slowing the user's breathing and thereby reducing stress. The breathing exercise may include or may be based on the target respiration information. The breathing exercise may be accessed from a library of breathing exercises such as on memory device 26. The breathing exercise may therefore be based on or may include predefined templates to assist the user in recovering from stress.

Figure 6 is an example user interface by which a breathing exercise is provided to a user. The user interface may be generated by apparatus 25 according to example embodiments. The curve 600 represents the user's detected respiration information, and 602 represents target respiration information or the breathing exercise. The respiration information 600 and 602 is plotted to illustrate amplitude of the breathing and/or target respiration. The user may therefore view the user interface and aim to inhale for the duration of 4 seconds, hold their breath for 4 seconds, and exhale for 6 seconds. The user interface shows that the user is breathing at a slightly faster rate than the target respiration rate, and the user is not holding their breath for the recommended duration. The user can, however, view the user interface and aim to slow their breathing to more closely match the target respiration information or breathing exercise.

The apparatus 25 may cause provision of the inhale and exhale duration precisely for each breath providing statistics as the user transitions from a regular breathing pattern (symmetrical inhale and exhale pattern) to a relaxed breathing pattern (characterized by asymmetrical breathing including a short inhale and longer exhale).

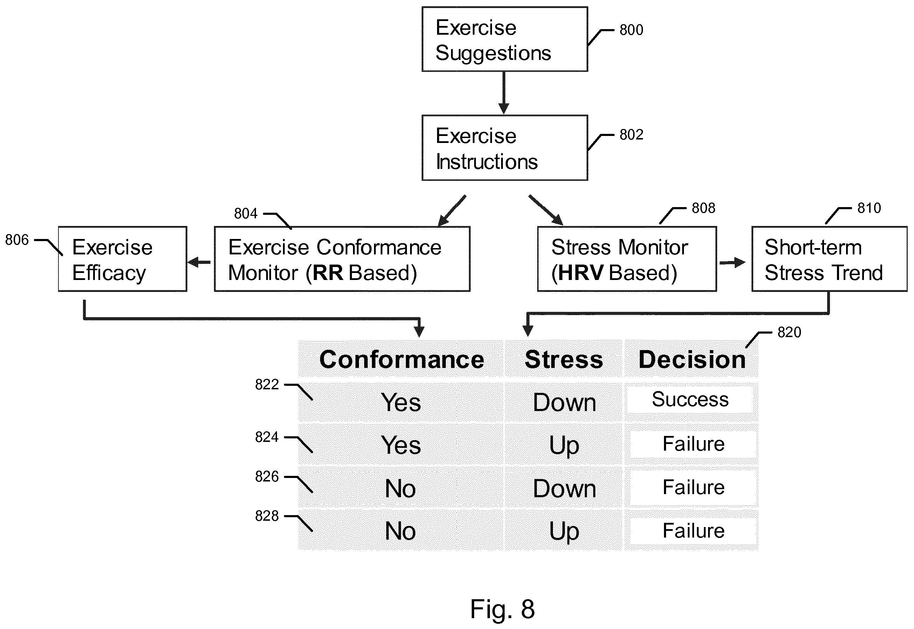

A user interface provided by certain example embodiments will depict the expected and performed breathing exercise on a display of a user device (e.g., mobile device, smartphone, and/or the like). While the subject is performing the breathing exercise, which may be tailored to abdomen breathing, the system will compare their breathing pattern with the target reparation information and/or breathing template. The efficacy of the breathing exercise is computed as the mean squared error between the two signals of the user respiration information and target respiration information, described in further detail below. In some examples, the user may not perform well due to person variability. Therefore, example embodiments will coach the subject to gradually achieve the desired breathing pattern. This is described in further detail with respect to Figure 8 below.

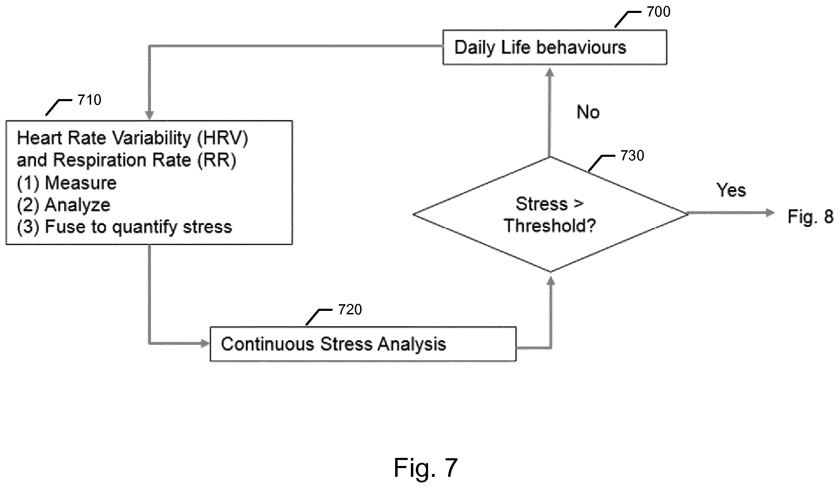

Figure 7 illustrates operations for managing stress that may be performed in accordance with example embodiments of apparatus 25. In this regard, apparatus 25 may be configured for assisting a user in measuring and managing stress in daily life, using respiration and/or heart rate information. In operation 700, apparatus 25 may include means, such as the processor 20, communication interface 24, or the like, for monitoring daily life behaviours. The daily life behaviors may include general activity and/or fitness level, and/or any other behaviours of the user. In operation 710, apparatus 25 may include means, such as the processor 20 or the like for monitoring and analyzing heart rate variability (HRV) and respiration rate (RR). Operation 710 may include measuring and analyzing HRV and/or RR. Operation 710 may further including fusing the HRV and RR so as to quantify stress. In some embodiments, operation 710 may include any of the operations described above with respect to Figure 3.

Therefore, in operation 720, apparatus 25 may include means, such as the processor 20, communication interface 24, or the like for providing continuous stress analysis. For example, apparatus 25 may be implemented in or communicatively connected to a wearable device and/or in a device affixed to a desktop or workstation, such that apparatus 25 may provide continuous or near-continuous and passive monitoring of a user's stress level. The monitoring may be considered passive in that the user may not necessarily be aware of the monitoring unless a threshold stress level is reached, as described in further detail below.

As shown in operation 730, apparatus 25 may include means, such as the processor 20 or the like, for determining whether a stress threshold has been satisfied. For example, the apparatus 25 may determine whether a predetermined threshold (e.g., a quantifiable measurement of stress) is met or exceeded. In an instance in which the threshold is satisfied, (e.g., "Yes"), example embodiments may proceed in performing operations provided in Figure 8, described below. In an instance in which the threshold is not satisfied (e.g., "No"), example embodiments may continue to monitor daily life behaviors as in operation 700. The operations of Figure 7 may be repeated accordingly. In this regard, example embodiments may provide ongoing or continuous monitoring of stress levels and alert a user accordingly and/or provide recommended breathing exercises as described with respect to Figure 8.

Figure 8 illustrates operations for providing a breathing exercise to a user and measuring the breathing exercise efficacy. In operation 800, the apparatus 25 may include means, such as the processor 20, user interface 22, or the like, for providing exercise suggestions. A user may select an exercise, and at operation 802, the apparatus 25 may include means, such as the processor 20, user interface 22, or the like for providing the exercise instructions relating to the selected exercise. The instructions may comprise a written narrative or a visual graph of breathing information such as that provided in Figure 6.

In operation 804, the apparatus 25 may include means, such as the processor 20, memory device 26, or the like, for providing an exercise conformance monitor based on respiratory rate (RR). As illustrated in the user interface of Figure 6, the apparatus 25 is configured to determine and monitor the difference between the user's respiration information and the target respiration information.

In operation 806, the apparatus 25 may include means, such as the processor 20 or the like, for determining the efficacy of the exercise. In this regard, the apparatus 25 may continue to monitor the user's breathing. If the difference between the user's respiration information and the target respiration information, narrows, the apparatus 25 may determine that the breathing exercise is effective. If the difference between the user respiration information and the target respiration information, stays constant or increases, the apparatus 25 may determine that the breathing exercise is ineffective and may suggest a different breathing exercise.

In some examples, the efficacy of the breathing exercise may be calculated as the mean square error (MSE) of the user respiration information to the target respiration information. In this regard, the efficacy may be calculated or quantified accordingly.

In certain embodiments, the efficacy of the breathing exercise may be calculated based on the phase difference of the chest breathing information and abdomen breathing information.

In operation 808, the apparatus 25 may include means, such as the processor 20 or the like, for providing a stress monitor that is HRV based. In this regard, the apparatus 25 may measure or estimate stress based on heart rate, or by calibrating the target respiration information based on HRV as described herein. In operation 810, the apparatus 25 may include means, such as the processor 20 or the like, for analyzing the short-term stress trend of the user. Changes in stress level may be determined and monitored by apparatus 25 accordingly.

The data generated or analyzed in operations 804, 806, 808 and 810 may be further processed by apparatus 25 to determine the example breathing exercise efficacy information in box 820. Row 822 indicates that conformance with the breathing exercise has occurred, and the stress level of the user has been determined to decrease. The decision or determination of success indicates the breathing exercise is effective. When the breathing exercise is determined as effective, the apparatus 25 may stop the provision of feedback and/or the breathing exercise and return to passive (e.g., in the background) monitoring of breathing and stress.

Row 822 indicates that conformance with the breathing exercise has occurred, but the stress level has gone up. In this regard, apparatus 25 may determine that the breathing exercise is not effective. Apparatus 25 may repeat any of the operations by returning to operation 800 and providing different exercise suggestions, and the process may continue.

Rows 826 and 828 indicate non-conformance with the breathing exercise, but respective decreased and increased stress. In either scenario, apparatus 25 may determine the breathing exercise to be ineffective as the user was unable to alter their breathing to match the target respiration provided by the exercise. In the failure scenarios, the apparatus 25 may return to operation 800 and provide different exercise suggestions, and the process may continue.

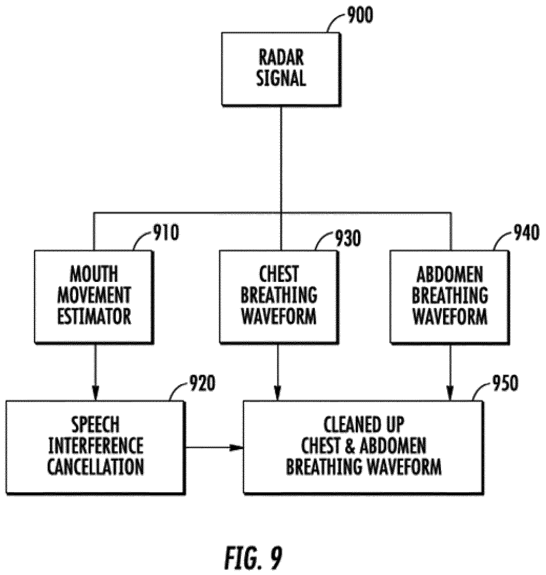

Figure 9 is a flowchart illustrating a flow of data in accordance with example embodiments of the present disclosure. The radar signal 900 may be processed by mouth movement estimator 910 to perform speech interference cancellation 920. In this regard, when apparatus 25 determines the user is speaking, variances caused by the speech may be removed from other data, such as but not limited to the chest breathing waveform 930 and abdomen breathing waveform 940. The radar signal 900 may be further processed to determine the chest breathing waveform 930 and abdomen breathing waveform 940. The apparatus 25 may then generate the cleaned up chest and abdomen breathing waveform 950.

Example embodiments therefore provide for determining stress recovery level based on chest and abdominal breathing information extracted from a radar signal. The target respiration information may be determined and/or calibrated for the particular user, such as based on HRV. Example embodiments may monitor the breathing of a user, and in some instances may do so passively in the background without interfering with the use of a device. However in certain embodiments, when the user's breathing is determined to be indicative of stress, feedback to the user may be provided. Example embodiments may provide a breathing exercise to the user to assist in steadying their breathing and reducing stress. Efficacy of the breathing exercise may then be determined and further feedback may be provided to the user.

The example embodiments described herein may therefore provide a technical effect of detecting stress in a user that may increase the effectiveness of activity monitoring and guidance in fields such as digital health and awareness. As such, the method and apparatus 25 of an example embodiment may provide technical advantages relating to efficiently, accurately and timely identifying states of user stress so as to alert the user to take corrective action.

As described above, Figures 3, 7, 8 and 9 illustrate flowcharts of an apparatus 25, method, and computer program product according to example embodiments of the disclosure. It will be understood that each block of the flowchart, and combinations of blocks in the flowchart, may be implemented by various means, such as hardware, firmware, processor, circuitry, and/or other devices associated with execution of software including one or more computer program instructions. For example, one or more of the procedures described above may be embodied by computer program instructions. In this regard, the computer program instructions which embody the procedures described above may be stored by a memory device 26 of an apparatus 25 employing an embodiment of the present disclosure and executed by a processor 20 of the apparatus 25. As will be appreciated, any such computer program instructions may be loaded onto a computer or other programmable apparatus (e.g., hardware) to produce a machine, such that the resulting computer or other programmable apparatus implements the functions specified in the flowchart blocks. These computer program instructions may also be stored in a computer-readable memory that may direct a computer or other programmable apparatus to function in a particular manner, such that the instructions stored in the computer-readable memory produce an article of manufacture, the execution of which implements the function specified in the flowchart blocks. The computer program instructions may also be loaded onto a computer or other programmable apparatus to cause a series of operations to be performed on the computer or other programmable apparatus to produce a computer-implemented process such that the instructions which execute on the computer or other programmable apparatus provide operations for implementing the functions specified in the flowchart blocks.

Accordingly, blocks of the flowchart support combinations of means for performing the specified functions and combinations of operations for performing the specified functions for performing the specified functions. It will also be understood that one or more blocks of the flowchart, and combinations of blocks in the flowchart, may be implemented by special purpose hardware-based computer systems which perform the specified functions, or combinations of special purpose hardware and computer instructions.

In some embodiments, certain ones of the operations above may be modified or further amplified. Furthermore, in some embodiments, additional optional operations may be included. Modifications, additions, or amplifications to the operations above may be performed in any order and in any combination.

Many modifications and other embodiments of the disclosures set forth herein will come to mind to one skilled in the art to which these disclosures pertain having the benefit of the teachings presented in the foregoing descriptions and the associated drawings. Therefore, it is to be understood that the disclosures are not to be limited to the specific embodiments disclosed and that modifications and other embodiments are intended to be included within the scope of the present disclosure. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the present disclosure. In this regard, for example, different combinations of elements and/or functions than those explicitly described above are also contemplated as may be set forth in some of the present disclosure. Although specific terms are employed herein, they are used in a generic and descriptive sense only and not for purposes of limitation. The scope of protection is solely defined by the appended claims.

**Claims**

1.  A method comprising:

    receiving user respiration and heart rate information according to a radar signal detected from a non-contact radar detection device (200); extracting chest breathing information and abdominal breathing information from the user respiration information; associating a phase difference between the chest breathing information and abdominal breathing information with a stress level indicator to determine stress recovery, wherein the stress level indicator comprises heart rate variability detected by the radar signal; and calibrating target respiration information based on the stress level indicator.

2.  The method of claim 1, further comprising:

    estimating mouth movement data according to the radar signal; and refining the user respiration information based on the mouth movement data.

3.  The method of any of claims 1-2, further comprising: identifying data provided according to the radar signal as attributed to speech.

4.  The method of any of claims 1-3, further comprising:

    comparing the user respiration information to target respiration information; based on the comparison of the user respiration information and the target respiration information, determining a difference between the user respiration information and the target respiration information; and in response to determining the difference, causing provision of feedback via a user interface.

5.  The method of any of claims 1-4, wherein the phase difference between the user respiration information and the target respiration information is determined as indicative of stress recovery.

6.  The method of any of claims 1-5, further comprising: identifying target respiration information for a particular user based on previously received user respiration information relating to the particular user.

7.  The method of any of claims 1-6, further comprising: determining target respiration information from a library of respiration information.

8.  The method of any of claims 1-7, further comprising:

    causing provision of information relating to a breathing exercise; determining an efficacy of the breathing exercise by monitoring the user respiration information; and causing provision of feedback relating to the efficacy of the breathing exercise.

9.  An apparatus (25) comprising at least one non-contact radar signal detection device (200), at least one processor (20), and at least one memory (26) including computer program code, the at least one memory (26) and the computer program code configured to, with the processor (20), cause the apparatus (25) to at least:

    generate user respiration and heart rate information according to a signal detected from the non-contact radar signal detection device (200); generate chest breathing information and abdominal breathing information from the use respiration information; associate a phase difference between the chest breathing information and abdominal breathing information with a stress level indicator to determine stress recovery, wherein the stress level indicator comprises heart rate variability detected by the radar signal; and calibrating target respiration information based on the stress level indicator.

10. The apparatus (25) of claim 9, wherein the signal detection device (200) comprises a radar detection device (200) configured to:

    emit a radar signal with a transmitter; and with a transceiver, detect the radar signal reflected from a body, wherein the user respiration information is generated according to the reflected radar signal.

11. The apparatus (25) of any of claims 9 or 10, wherein the at least one memory (26) and the computer program code are further configured to:

    estimate mouth movement data according to the signal; and

refine the user respiration information based on the mouth movement data.

12. The apparatus (25) of any of claims 9-11, wherein the at least one memory (26) and the computer program code are further configured to, with the processor (20), cause the apparatus (25) to at least:

compare the user respiration information to target respiration information;
based on the comparison of the user respiration information and the target respiration information, determine a difference between the user respiration information and the target respiration information; and
in response to determining the difference, causing provision of feedback via a user interface.

13. The apparatus (25) of any of claims 9 - 12, wherein the at least one memory (26) and the computer program code are further configured to, with the processor (20), cause the apparatus (25) to at least:

cause provision of information relating to a breathing exercise;
determine an efficacy of the breathing exercise by monitoring the user respiration information; and
cause provision of feedback relating to the efficacy of the breathing exercise.

14. A computer program product comprising at least one non-transitory computer-readable storage medium having computer-executable program code instructions stored therein, the computer-executable program code instructions comprising program code instructions to at least:

receive user respiration and heart rate information according to a radar signal detected from a non-contact radar detection device (200);
extract chest breathing information and abdominal breathing information from the user respiration information;
associating a phase difference between the chest breathing information and abdominal breathing information with a stress indicator to determine stress recovery, wherein the stress level indicator comprises heart rate variability detected by the radar signal; and
calibrating target respiration information based on the stress level indicator.

15. The computer program product of claim 14, wherein the computer-executable program code instructions comprising program code instructions to at least:

estimate mouth movement data according to the radar signal; and
refine the user respiration information based on the mouth movement data.

**Patentansprüche**

1. Verfahren, das Folgendes umfasst:

Empfangen von Benutzeratmungs- und Herzfrequenzinformationen gemäß einem Radarsignal, das von einer kontaktlosen Radardetektionsvorrichtung (200) detektiert wird;
Extrahieren von Brustatmungsinformationen und Bauchatmungsinformationen aus den Benutzeratmungsinformationen;
Verknüpfen einer Phasendifferenz zwischen den Brustatmungsinformationen und den Bauchatmungsinformationen mit einem Stresspegelindikator, um eine Stresswiederherstellung zu bestimmen, wobei der Stresspegelindikator eine Herzfrequenzvariabilität umfasst, die vom Radarsignal detektiert wird; und
Kalibrieren von Zielatmungsinformationen auf Basis des Stresspegelindikators.

2. Verfahren nach Anspruch 1, das ferner Folgendes umfasst:

Schätzen von Mundbewegungsdaten gemäß dem Radarsignal; und
Verfeinern von Benutzeratmungsinformationen auf Basis der Mundbewegungsdaten.

3. Verfahren nach einem der Ansprüche 1 bis 2, das ferner Folgendes umfasst:
Identifizieren von Daten, die gemäß dem Radarsignal bereitgestellt werden, die Sprache zugewiesen werden.

4. Verfahren nach einem der Ansprüche 1 bis 3, das ferner Folgendes umfasst:

Vergleichen der Benutzeratmungsinformationen mit Zielatmungsinformationen;
Bestimmen einer Differenz zwischen den Benutzeratmungsinformationen und den Zielatmungsinformationen auf Basis des Vergleichs der Benutzeratmungsinformationen und der Zielatmungsinformationen; und
Veranlassen einer Bereitstellung einer Rückmeldung via eine Benutzerschnittstelle in Reaktion auf das Bestimmen der Differenz.

5. Verfahren nach einem der Ansprüche 1 bis 4, wobei die Phasendifferenz zwischen den Benutzeratmungsinformationen und den Zielatmungsinformationen als Anzeige einer Stresswiederherstellung bestimmt wird.

6. Verfahren nach einem der Ansprüche 1 bis 5, das ferner Folgendes umfasst:
Identifizieren von Zielatmungsinformationen für einen bestimmten Benutzer auf Basis von zuvor empfangenen Benutzeratmungsinformationen, die sich auf den bestimmten Benutzer beziehen.

7. Verfahren nach einem der Ansprüche 1 bis 6, das ferner Folgendes umfasst:
Bestimmen von Zielatmungsinformationen anhand einer Bibliothek von Atmungsinformationen.

8. Verfahren nach einem der Ansprüche 1 bis 7, das ferner Folgendes umfasst:

Veranlassen einer Bereitstellung von Informationen, die sich auf eine Atemübung beziehen;
Bestimmen einer Effizienz der Atemübung durch Überwachen der Benutzeratmungsinformationen; und
Veranlassen einer Bereitstellung einer Rückmeldung, die sich auf die Effizienz der Atemübung bezieht.

9. Einrichtung (25), die mindestens eine kontaktlose Radarsignaldetektionsvorrichtung (200), mindestens einen Prozessor (20) und mindestens einen Speicher (26), der einen Computerprogrammcode beinhaltet, umfasst, wobei der mindestens eine Speicher (26) und der Computerprogrammcode dazu ausgelegt sind, die Einrichtung (25) mit dem Prozessor (20) zu veranlassen, mindestens Folgendes durchzuführen:

Erzeugen von Benutzeratmungs- und Herzfrequenzinformationen gemäß einem Signal, das von der kontaktlosen Radarsignaldetektionsvorrichtung (200) detektiert wird;
Erzeugen von Brustatmungsinformationen und Bauchatmungsinformationen aus den Benutzeratmungsinformationen;
Verknüpfen einer Phasendifferenz zwischen den Brustatmungsinformationen und den Bauchatmungsinformationen mit einem Stresspegelindikator, um eine Stresswiederherstellung zu bestimmen, wobei der Stresspegelindikator eine Herzfrequenzvariabilität umfasst, die vom Radarsignal detektiert wird; und
Kalibrieren von Zielatmungsinformationen auf Basis des Stresspegelindikators.

10. Einrichtung (25) nach Anspruch 9, wobei die Signaldetektionsvorrichtung (200) eine Radardetektionsvorrichtung (200) umfasst, die zu Folgendem ausgelegt ist:

Emittieren eines Radarsignals mit einem Sender; und
Detektieren des Radarsignals, das von einem Körper reflektiert wird, mit einem Sendeempfänger, wobei die Benutzeratmungsinformationen gemäß dem reflektierten Radarsignal erzeugt werden.

11. Einrichtung (25) nach einem der Ansprüche 9 oder 10, wobei der mindestens eine Speicher (26) und der Computerprogrammcode ferner zu Folgendem ausgelegt sind:

Schätzen von Mundbewegungsdaten gemäß dem Signal; und
Verfeinern von Benutzeratmungsinformationen auf Basis der Mundbewegungsdaten.

12. Einrichtung (25) nach einem der Ansprüche 9 bis 11, wobei der mindestens eine Speicher (26) und der Computerprogrammcode ferner dazu ausgelegt sind, die Einrichtung (25) mit dem mindestens einen Prozessor (20) mindestens zu Folgendem zu veranlassen:

Vergleichen der Benutzeratmungsinformationen mit Zielatmungsinformationen;
Bestimmen einer Differenz zwischen den Benutzeratmungsinformationen und den Zielatmungsinformationen auf Basis des Vergleichs der Benutzeratmungsinformationen und der Zielatmungsinformationen; und
Veranlassen einer Bereitstellung einer Rückmeldung via eine Benutzerschnittstelle in Reaktion auf das Bestimmen der Differenz.

13. Einrichtung (25) nach einem der Ansprüche 9 bis 12, wobei der mindestens eine Speicher (26) und der Computerprogrammcode ferner dazu ausgelegt sind, die Einrichtung (25) mit dem mindestens einen Prozessor (20) mindestens zu Folgendem zu veranlassen:

Veranlassen einer Bereitstellung von Informationen, die sich auf eine Atemübung beziehen;
Bestimmen einer Effizienz der Atemübung durch Überwachen der Benutzeratmungsinformationen; und
Veranlassen einer Bereitstellung einer Rückmeldung, die sich auf die Effizienz der Atemübung bezieht.

14. Computerprogrammprodukt, das mindestens ein nichttransitorisches computerlesbares Speichermedium umfasst, in dem computerausführbare Programmcodeanweisungen gespeichert sind, wobei die computerausführbaren Programmcodeanweisungen Programmcodeanweisungen für mindestens Folgendes umfassen:

Empfangen von Benutzeratmungs- und Herzfrequenzinformationen gemäß einem Radarsignal, das von einer kontaktlosen Radardetektionsvorrichtung (200) detektiert wird; Extrahieren von Brustatmungsinformationen und Bauchatmungsinformationen aus den Benutzeratmungsinformationen; Verknüpfen einer Phasendifferenz zwischen den Brustatmungsinformationen und den Bauchatmungsinformationen mit einem Stressindikator, um eine Stresswiederherstellung zu bestimmen, wobei der Stresspegelindikator eine Herzfrequenzvariabilität umfasst, die vom Radarsignal detektiert wird; und Kalibrieren von Zielatmungsinformationen auf Basis des Stresspegelindikators.

15. Computerprogrammprodukt nach Anspruch 14, wobei die computerausführbaren Programmcodeanweisungen Programmcodeanweisungen für mindestens Folgendes umfassen:

Schätzen von Mundbewegungsdaten gemäß dem Radarsignal; und Verfeinern von Benutzeratmungsinformationen auf Basis der Mundbewegungsdaten.

## Revendications

1. Procédé comprenant les étapes suivantes :

recevoir des informations de respiration et de fréquence cardiaque d'utilisateur selon un signal radar détecté par un dispositif de détection radar sans contact (200) ; extraire des informations de respiration thoracique et des informations de respiration abdominale à partir des informations de respiration d'utilisateur ; associer une différence de phase entre les informations de respiration thoracique et les informations de respiration abdominale à un indicateur de niveau d'effort afin de déterminer une récupération de l'effort, dans lequel l'indicateur de niveau d'effort comprend une variabilité de fréquence cardiaque détectée par le signal radar ; et étalonner des informations de respiration cible sur la base de l'indicateur de niveau d'effort.

2. Procédé selon la revendication 1, comprenant en outre les étapes suivantes :

estimer des données de mouvements de bouche selon le signal radar ; et

affiner les informations de respiration d'utilisateur sur la base des données de mouvements de bouche.

3. Procédé selon l'une des revendications 1 à 2, comprenant en outre l'étape suivante : identifier des données fournies selon le signal radar comme étant attribuées à la parole.

4. Procédé selon l'une des revendications 1 à 3, comprenant en outre les étapes suivantes :

comparer les informations de respiration d'utilisateur aux informations de respiration cible ; sur la base de la comparaison des informations de respiration d'utilisateur et des informations de respiration cible, déterminer une différence entre les informations de respiration d'utilisateur et les informations de respiration cible ; et en réponse à la détermination de la différence, provoquer la mise à disposition d'une rétroaction via une interface utilisateur.

5. Procédé selon l'une des revendications 1 à 4, dans lequel la différence de phase entre les informations de respiration d'utilisateur et les informations de respiration cible est déterminée comme indicative de la récupération de l'effort.

6. Procédé selon l'une des revendications 1 à 5, comprenant en outre l'étape suivante : identifier des informations de respiration cible pour un utilisateur particulier sur la base d'informations de respiration d'utilisateur précédemment reçues relatives à l'utilisateur particulier.

7. Procédé selon l'une des revendications 1 à 6, comprenant en outre l'étape suivante : déterminer des informations de respiration cible à partir d'une bibliothèque d'informations de respiration.

8. Procédé selon l'une des revendications 1 à 7, comprenant en outre les étapes suivantes :

provoquer la mise à disposition d'informations relatives à un exercice de respiration ; déterminer une efficacité de l'exercice de respiration en surveillant les informations de respiration d'utilisateur ; et provoquer la mise à disposition d'une rétroaction relative à l'efficacité de l'exercice de respiration.

9. Appareil (25) comprenant au moins un dispositif de détection de signal radar sans contact (200), au moins un processeur (20) et au moins une mémoire (26) comportant un code de programme informatique, la au moins une mémoire (26) et le code de programme informatique étant configurés pour, avec le processeur (20), amener l'appareil (25) à au moins :

> générer des informations de respiration et de fréquence cardiaque d'utilisateur selon un signal détecté par le dispositif de détection de signal radar sans contact (200) ;
>
> générer des informations de respiration thoracique et des informations de respiration abdominale à partir des informations de respiration d'utilisateur ;
>
> associer une différence de phase entre les informations de respiration thoracique et les informations de respiration abdominale à un indicateur de niveau d'effort afin de déterminer une récupération de l'effort, dans lequel l'indicateur de niveau d'effort comprend une variabilité de fréquence cardiaque détectée par le signal radar ; et
>
> étalonner des informations de respiration cible sur la base de l'indicateur de niveau d'effort.

10. Appareil (25) selon la revendication 9, dans lequel le dispositif de détection de signal (200) comprend un dispositif de détection radar (200) configuré pour :

> émettre un signal radar à l'aide d'un émetteur ; et
>
> à l'aide d'un émetteur-récepteur, détecter le signal radar réfléchi par un corps, dans lequel les informations de respiration d'utilisateur sont générées selon le signal radar réfléchi.

11. Appareil (25) selon l'une des revendications 9 ou 10, dans lequel la au moins une mémoire (26) et le code de programme informatique sont en outre configurés pour :

> estimer des données de mouvements de bouche selon le signal ; et
>
> affiner les informations de respiration d'utilisateur sur la base des données de mouvements de bouche.

12. Appareil (25) selon l'une des revendications 9 à 11, dans lequel la au moins une mémoire (26) et le code de programme informatique sont en outre configurés pour, avec le processeur (20), amener l'appareil (25) à au moins :

> comparer les informations de respiration d'utilisateur aux informations de respiration cible ;
>
> sur la base de la comparaison des informations de respiration d'utilisateur et des informations de respiration cible, déterminer une différence entre les informations de respiration d'utilisateur et les informations de respiration cible ; et

> en réponse à la détermination de la différence, provoquer la mise à disposition d'une rétroaction via une interface utilisateur.

13. Appareil (25) selon l'une des revendications 9 à 12, dans lequel la au moins une mémoire (26) et le code de programme informatique sont en outre configurés pour, avec le processeur (20), amener l'appareil (25) à au moins :

> provoquer la mise à disposition d'informations relatives à un exercice de respiration ;
>
> déterminer une efficacité de l'exercice de respiration en surveillant les informations de respiration d'utilisateur ; et
>
> provoquer la mise à disposition d'une rétroaction relative à l'efficacité de l'exercice de respiration.

14. Produit de programme informatique comprenant au moins un support de stockage non transitoire lisible par ordinateur dans lequel sont stockées des instructions de code de programme exécutables par ordinateur, les instructions de code de programme exécutables par ordinateur comprenant des instructions de code de programme pour au moins :

> recevoir des informations de respiration et de fréquence cardiaque d'utilisateur selon un signal radar détecté par un dispositif de détection radar sans contact (200) ;
>
> extraire des informations de respiration thoracique et des informations de respiration abdominale à partir des informations de respiration d'utilisateur ;
>
> associer une différence de phase entre les informations de respiration thoracique et les informations de respiration abdominale à un indicateur d'effort pour déterminer une récupération de l'effort, dans lequel l'indicateur de niveau d'effort comprend une variabilité de fréquence cardiaque détectée par le signal radar ; et
>
> étalonner des informations de respiration cible sur la base de l'indicateur de niveau d'effort.

15. Produit de programme informatique selon la revendication 14, dans lequel les instructions de code de programme exécutables par ordinateur comprennent des instructions de code de programme pour au moins :

> estimer des données de mouvements de bouche selon le signal radar ; et
>
> affiner les informations de respiration d'utilisateur sur la base des données de mouvements de bouche.

Fig. 1

Fig. 2

Receive user respiration information according to a radar signal detected from a radar detection device — 300

Estimate mouth movement data according to the radar signal — 302

Refine the user respiration information based on the mouth movement data — 304

Extract chest breathing information and abdominal breathing information from the user respiration information — 306

Associating a phase difference between the chest breathing information and abdominal breathing information with a stress level indicator to determine stress recovery — 308

Calibrate target respiration information — 310

Fig. 3

Fig. 4

Compare the user respiration information to target respiration information ⌐ 500

Based on the comparison of the user respiration information and the target respiration information, determine a difference between the user respiration information and the target respiration information ⌐ 502

In response to determining the difference, cause provision of feedback via a user interface ⌐ 504

Fig. 5

Inhale ⋮ Hold ⋮ Exhale

600

602

Amplitude

4s 4s 6s

Time

Fig. 6

Fig. 7

EP 3 384 843 B1

Fig. 8

FIG. 9

EP 3 384 843 B1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011060215 A
- WO 2104055755 A

- US 2010152600 A